# FASCICULE DE BREVET EUROPEEN

(11) **EP 0 770 381 B1**
(45) Date de publication et mention de la délivrance du brevet: **18.02.1998**
(21) Numéro de dépôt: 96402054.9
(22) Date de dépôt: 26.09.1996
(51) Int. Cl.: A61K 7/48

(54) **Support, et composition contenant ce support et un actif cosmétique ou dermatologique stabilisé**
Träger und Zusammensetzung, die diesen Träger und einen stabilisierten kosmetischen oder dermatologischen Wirkstoff enthält
Support and composition containing it together with a stabilised cosmetic or dermatologic agent

(30) Priorité: 23.10.1995 FR 9512446
(43) Date de publication de la demande: 02.05.1997
(73) Titulaire: L'OREAL, 75008 Paris (FR)
(72) Inventeur: De Salvert, Armelle, 75013 Paris (FR)
(74) Mandataire: Dodin, Catherine

(56) Documents cités:
- EP-A- 0 404 532
- EP-A- 0 437 956
- EP-A- 0 623 338
- US-A- 4 673 569

## Description

La présente invention se rapporte à un support formant un milieu stable bien qu'exempt de tensioactif et de stabilisant, susceptible d'être utilisé dans une composition contenant un actif sensible aux facteurs extérieurs et/ou à l'eau, et à l'utilisation d'une telle composition pour le traitement cosmétique et/ou dermatologique de la peau, y compris du cuir chevelu.

Il est connu d'introduire dans les compositions cosmétiques et/ou dermatologiques des actifs en vue d'apporter des traitements spécifiques à la peau, par exemple pour lutter contre le dessèchement, le vieillissement ou la pigmentation de la peau, pour traiter l'acné ou certaines maladies de la peau (eczéma, psoriasis), pour combattre les surcharges pondérales, pour favoriser la restructuration de la peau ou son renouvellement cellulaire, ou aussi pour colorer la peau.

Par exemple, l'acide ascorbique (ou vitamine C) est connu pour stimuler la croissance du tissu conjonctif, et notamment celle du collagène. Il permet aussi de renforcer les défenses du tissu cutané contre les agressions extérieures telles que le rayonnement ultraviolet ou la pollution. Il est également utilisé pour enlever les taches et la pigmentation de la peau, et aussi pour favoriser la cicatrisation de la peau.

Il est connu aussi que l'application de rétinol ou vitamine A permet de lutter notamment contre le vieillissement cutané et contre certains désordres de la peau tels que l'acné ou les troubles de kératinisation ou de cicatrisation.

Par ailleurs, l'art antérieur enseigne depuis de longues années l'implication de la dihydroxyacétone dans la coloration artificielle de la peau (Bobin et al. J. Soc. Cosmet. Chem., 35 pages 265-272, 1984). La dihydroxyacétone réagit avec les acides aminés naturellement contenus dans le film lipidique du *stratum corneum* et par une réaction de Maillard forme des mélanoïdes (Maillard L.C., C.R. Acad. Sci. 154, 66-68, 1912). L'application de la dihydroxyacétone sur la peau permet donc de conférer à celle-ci l'apparence d'une peau bronzée, sans avoir les inconvénients (brûlures, risques de cancer) rencontrés lors d'une exposition au soleil.

En outre, il est connu également d'introduire dans les compositions cosmétiques des enzymes, et notamment des protéases et des lipases utilisées pour leurs propriétés protéolytiques et lipolytiques. Ces enzymes sont recherchées dans le domaine cosmétique pour leur pouvoir lissant et nettoyant, et leur aptitude à éliminer les cellules mortes de la peau.

Malheureusement, certains actifs, et en particulier ceux cités ci-dessus, sont instables du fait de leur sensibilité à des facteurs extérieurs tels que la lumière, la chaleur ou la présence d'oxygène soit de l'air soit contenu dans l'eau. Ainsi, la stabilité de la dihydroxyacétone, de la vitamine C, de la vitamine A ou encore des enzymes dans une composition est tout à fait relative : ces actifs dans une composition se dégradent au cours du temps.

Cette instabilité va à l'encontre de l'efficacité recherchée et peut, de plus, être source de désagréments pour l'utilisateur, par exemple lorsque l'instabilité de l'actif entraîne des modifications de couleur et/ou d'odeur de la composition le contenant.

Aussi, différents moyens ont été envisagés pour stabiliser ces actifs. Lorsque l'actif comporte un site réactif, notamment dans le cas des vitamines et de la dihydroxyacétone, un des moyens pour le stabiliser consiste par exemple à bloquer ce site par estérification avec notamment des dérivés phosphatés, sulfatés ou alkylés et à utiliser ces dérivés à la place de l'actif libre. Malheureusement, ces dérivés présentent une efficacité moins bonne que l'actif libre.

Il a été aussi envisagé d'utiliser des précurseurs de tels actifs, qui, après application sur la peau, sont coupés par les enzymes cutanées et libèrent alors l'actif libre (voir le document EP-A-487404). Mais l'utilisation de tels dérivés ne permet pas toujours une libération rapide et en quantité suffisante d'actif à la surface de la peau.

En outre, il a été envisagé de mettre un actif, notamment une enzyme dans une composition pulvérulente (voir le document JP-A-63-130514). Il a été aussi envisagé d'utiliser ces actifs, et notamment les enzymes, sous forme immobilisée sur des supports polymériques (voir le document JP-A-61-207499) ou dans des microcapsules (voir le document JP-A-61-254244). Malheureusement, ces moyens nécessitent une mise en oeuvre particulière, ce qui accroît le coût et le temps de préparation de la composition.

Lorsque les actifs sont sensibles à l'eau, une autre solution consiste à les incorporer dans un milieu liquide anhydre (voir le document US-A-5322683). Malheureusement, cette solution limite la forme galénique de la composition et ne permet pas l'incorporation d'actifs hydrophiles.

Il subsiste donc le besoin d'une composition dans laquelle des actifs cosmétiques et/ou dermatologiques sensibles conserveraient toutes leurs propriétés et donc leur efficacité au cours du temps.

La demanderesse a maintenant trouvé de manière inattendue qu'un support comprenant au plus 10 % d'eau, une huile amphiphile, un glycol en C₂ à C₄ ou un dérivé éther-oxyde d'un glycol en C₂ à C₄, en mélange avec un alcool primaire et/ou un glycol en C₅ à C₇, était susceptible de maintenir l'activité d'un actif sensible aux facteurs extérieurs et/ou à l'eau, et d'éviter la dégradation de cet actif.

La présente invention a donc pour objet un support, caractérisé en ce qu'il comprend au plus 10 % en poids d'eau, au moins une huile amphiphile, au moins un polyol ou dérivé de polyol choisi parmi les glycols en C₂ à C₄, les dérivés éther-oxydes d'un glycol en C₂ à C₄ et leurs mélanges, et au moins un solvant de l'huile et de l'eau, à fonction alcool et en ce qu'il est exempt de tensioactif.

L'eau dans le support selon l'invention est présente de préférence en une quantité allant de 1 à 10 % en poids par rapport au poids total de la composition.

Le solvant à fonction alcool peut être notamment un alcool primaire en C₂ à C₈, un glycol en C₅ à C₇ ou un mélange d'alcool primaire et de glycol en C₅ à C₇.

Bien qu'exempt de tensioactif et de stabilisant, le support obtenu selon l'invention se présente sous forme d'un fluide transparent ou translucide, présentant une bonne stabilité. L'absence de tensioactif a l'avantage de rendre le support moins irritant.

Selon l'invention, on entend par 〈〈 huile amphiphile 〉〉 une huile ayant des affinités avec l'eau. Il peut s'agir notamment d'esters ou d'éthers comportant un atome d'oxygène ayant des affinités avec l'eau et ayant un HLB (Hydrophilic Lipophilic Balance) de 6 à 12, et de préférence un HLB d'environ 10. On peut citer en particulier comme huile amphiphile utilisable dans la présente invention le benzoate de laureth-2, le benzoate de glycereth-7, le dioctanoate/diisononanoate de diéthylène glycol, l'éther stéarylique de polyoxypropylène-15, le malate d'éthyl-2-hexyle, l'adipate d'isopropyle, le copolymère de PPG-7 et d'acide succinique, le dioctanoate de néopentylglycol.

L'huile amphiphile est de préférence présente dans le support de l'invention en une quantité allant de 10 à 55 % en poids, et plus préférentiellement de 20 à 30 % en poids par rapport au poids total du support.

Comme glycols en C₂ à C₄ utilisables selon l'invention, on peut citer notamment le propylène glycol et le butylène glycol. Par ailleurs, on entend par 〈〈 dérivé éther-oxyde d'un glycol en C₂ à C₄ 〉〉 les glycols obtenus par condensation de deux glycols en C₂ à C₄ avec formation d'une liaison éther, tels que le dipropylène glycol, et leurs dérivés tels que l'éthoxydiglycol vendu sous le nom de 〈〈 Transcutol 〉〉 par la société Gattefosse.

Comme glycols en C₅ à C₇ utilisables selon l'invention, on peut citer notamment le pentanediol-1,2 et plus particulièrement celui vendu sous la dénomination 〈〈 Hydrolite-5 〉〉 par la société Dragoco.

L'alcool primaire utilisable dans le support de l'invention peut être notamment l'alcool éthylique.

Le support selon l'invention peut contenir de 10 à 20 % en poids d'un ou plusieurs glycols en C₂ à C₄ et/ou d'un ou plusieurs dérivés éther-oxyde d'un glycol en C₂ à C₄, et préférentiellement de 15 à 18 % en poids par rapport au poids total du support.

Par ailleurs, le support selon l'invention peut contenir de 5 à 60 % en poids, et préférentiellement de 15 à 55 % en poids d'un ou plusieurs alcools primaires et/ou d'un ou plusieurs glycols en C₅ à C₇. La quantité de glycol en C₅ à C₇ va de manière avantageuse de 5 à 10 % en poids quand le support contient un alcool primaire.

De façon avantageuse, le support de l'invention peut être utilisé dans une composition à action topique contenant des actifs sensibles aux facteurs extérieurs tels que la lumière et la chaleur, et/ou à l'eau. De façon surprenante, ces actifs restent stables dans la composition selon l'invention.

Auusi, la présente invention a aussi pour objet une composition contenant un actif à action topique, sensible aux facteurs extérieurs et/ou à l'eau, caractérisée en.ce qu'elle contient un support tel que défini ci-dessus.

Les actifs à action topique, sensibles aux facteurs extérieurs et/ou à l'eau, auxquels peut s'appliquer l'invention peuvent être notamment les enzymes et les actifs comportant au moins une fonction hydroxyle.

Comme enzymes, on peut citer par exemple les lipases et les protéases. Parmi les protéases, on peut citer par exemple celle vendue sous la dénomination commerciale "Subtilisine SP 544" par la société Novo Nordisk et celle vendue sous la dénomination commerciale "Lysoveg" par la société Laboratoires Sérobiologiques de Nancy.

Comme actifs comportant au moins une fonction hydroxyle, on peut citer en particulier les vitamines estérifiables telles que le rétinol (vitamine A) et ses dérivés, l'acide ascorbique (vitamine C) et ses dérivés ; les cétones hydroxylées telles que la dihydroxyacétone.

Ces actifs sont avantageusement présents dans la composition selon l'invention en une quantité allant de 0,5 à 10 % en poids et plus particulièrement de 1 à 5 % en poids par rapport au poids total du composition.

Pour une application topique, la composition selon l'invention doit contenir un milieu topiquement acceptable, c'est-à-dire compatible avec la peau et/ou les cheveux et/ou les muqueuses.

La composition selon l'invention peut être utilisée, selon l'actif qu'elle contient, pour le traitement cosmétique et/ou dermatologique de la peau et/ou des cheveux.

La composition selon l'invention peut constituer notamment des compositions de nettoyage, de protection, de traitement ou de soin pour le visage, pour le cou, pour les mains ou pour le corps, des produits de bronzage artificiel, ou des produits pour les cheveux, et notamment pour le soin du cuir chevelu, par exemple sous forme de lotions traitantes.

Dans le cas particulier de l'acide ascorbique, la composition peut être destinée par exemple à la dépigmentation de la peau ou au traitement de l'acné. Ce dernier peut être également traité par une composition contenant du rétinol.

Aussi, l'invention a en outre pour objet l'utilisation de la composition telle que définie ci-dessus pour la dépigmentation de la peau, l'actif étant l'acide ascorbique.

L'invention a encore pour objet l'utilisation de la composition que définie ci-dessus pour préparer une pommade ou un onguent dermatologique destiné au traitement thérapeutique de l'acné, l'actif étant l'acide ascorbique et/ou le rétinol.

Quand elle contient de la dihydroxyacétone, la composition selon l'invention peut constituer une composition autobronzante.

Aussi, l'invention a encore pour objet l'utilisation de la composition telle que définie ci-dessus pour colorer la peau, l'actif étant la dihydroxyacétone.

La dihydroxyacétone est de préférence utilisée en une quantité allant de 1 à 8 % en poids, et mieux de 1 à 5 % en poids par rapport au poids total de la composition.

En outre, l'invention a aussi pour objet une composition autobronzante à base de dihydroxyacétone, caractérisée en ce qu'elle comprend au plus 10 % en poids d'eau, au moins une huile amphiphile, au moins un polyol ou dérivé de polyol choisi parmi les glycols en C₂ à C₄, les dérivés éther-oxydes d'un glycol en C₂ à C₄ et leurs mélanges, et au moins un solvant de l'huile et de l'eau, à fonction alcool.

De façon connue, la composition selon l'invention peut contenir également des adjuvants habituels dans le domaine cosmétique ou dermatologique, tels que les conservateurs, les antioxydants, les parfums, les filtres, les gélifiants, les séquestrants, les huiles essentielles, les matières colorantes, les actifs hydrophiles ou lipophiles autres que ceux indiqués ci-dessus.

Comme gélifiants, on peut citer par exemple les polysaccharides tels que les hydroxypropylcelluloses.

Comme actifs hydrophiles, on peut utiliser par exemple les protéines ou les hydrolysats de protéine et les acides aminés.

Comme actifs lipophiles, on peut utiliser par exemple le tocophérol (vitamine E) et ses dérivés, les acides gras essentiels, les céramides, les huiles essentielles.

Les quantités des différents constituants de la composition selon l'invention sont celles classiquement utilisées dans les domaines considérés.

Les exemples qui suivent sont donnés à titre illustratif afin de mieux faire comprendre l'invention. Les quantités indiquées sont des pourcentages en poids.

Dans tous les exemples, les compositions sont préparées de la manière suivante : si un gélifiant est présent, on le disperse à froid dans le solvant à fonction alcool (alcool éthylique) sous agitation constante, puis on ajoute les différents constituants les uns après les autres, après dilution préalable des actifs dans l'eau.

### Exemple 1 : Fluide autobronzant

| | |
|---|---|
| Alcool éthylique | 43,4 % |
| Hydroxypropylcellulose | 0,7 % |
| Vitamine E | 0,5 % |
| Butylène glycol | 10 % |
| Dipropylène glycol | 5 % |
| Benzoate de laureth-2 (Dermol 126 vendu par la société Alzo) | 12,5 % |
| Benzoate de glycereth-7 (Dermol G76 vendu par la société Alzo) | 7,5 % |
| Dioctanoate/diisononanoate de diéthylène glycol (Dermol 489 vendu par la société Alzo) | 5 % |
| Dihydroxyacétone | 5 % |
| Parfum | 0,4 % |
| Eau | 10 % |

Le fluide obtenu se présente sous forme d'un liquide transparent, stable dans le temps, agréable à appliquer. Il permet un bronzage régulier du visage et du corps par application quotidienne pendant au moins une semaine.

Un test d'utilisation a été réalisé sur un panel de 40 femmes qui ont appliqué le fluide autobronzant sur le visage pendant 10 jours.

Les utilisatrices ont apprécié la transparence du fluide et ont indiqué qu'il était facile à appliquer et particulièrement frais lors de l'application sur la peau. Après les 10 jours d'utilisation, elles ont jugé que le bronzage avait été obtenu plus rapidement qu'avec les compositions habituelles et que la teinte obtenue était bien uniforme. 73 % des utilisatrices se sont déclarées prêtes à l'acheter.

### Exemple 2 : Fluide autobronzant

| | |
|---|---|
| Alcool éthylique | 33,2 % |
| Hydroxypropylcellulose | 0,7 % |
| Vitamine E | 0,5 % |
| Butylène glycol | 10 % |
| Dipropylène glycol | 7,5 % |
| Benzoate de laureth-2 (Dermol 126 vendu par la société Alzo) | 10 % |
| Dioctanoate/diisononanoate de diéthylène glycol (Dermol 489 vendu par la société Alzo) | 8 % |
| Ether stéarylique de polyoxypropylène-15 (Arlamol E vendu par la société ICI) | 4,5 % |
| Pentane diol-1,2 (Hydrolite-5 vendu par la société Dragoco) | 10 % |
| Dihydroxyacétone | 5 % |
| Parfum | 0,6 % |
| Eau | 10 % |

Le fluide obtenu a les mêmes caractéristiques que celui de l'exemple 1.

### Exemple 3 : Fluide autobronzant

| | |
|---|---|
| Alcool éthylique | 53,8 % |
| Hydroxypropylcellulose | 0,5 % |
| Vitamine E | 0,5 % |
| Butylène glycol | 15 % |
| Dioctanoate/diisononanoate de diéthylène glycol (Dermol 489 vendu par la société Alzo) | 20 % |
| Dihydroxyacétone | 5 % |
| Parfum | 0,2 % |
| Eau | 5 % |

Le fluide obtenu a les mêmes caractéristiques que celui de l'exemple 1.

### Exemple 4 : Fluide autobronzant

| | |
|---|---|
| Alcool éthylique | 53,8 % |
| Hydroxypropylcellulose | 0,5 % |
| Butylène glycol | 15 % |
| Dioctanoate/diisononanoate de diéthylène glycol (Dermol 489 vendu par la société Alzo) | 15,5 % |
| Ether stéarylique de polyoxypropylène-15 (Arlamol E vendu par la société ICI) | 5 % |
| Dihydroxyacétone | 5 % |
| Parfum | 0,2 % |
| Eau | 5 % |

Le fluide obtenu a les mêmes caractéristiques que celui de l'exemple 1.

### Exemple 5 : Fluide autobronzant

| | |
|---|---|
| Alcool éthylique | 37,2 % |
| Hydroxypropylcellulose | 0,1 % |
| Butylène glycol | 15 % |
| Dioctanoate/diisononanoate de diéthylène glycol (Dermol 489 vendu par la société Alzo) | 30 % |
| Ether stéarylique de polyoxypropylène-15 (Arlamol E vendu par la société ICI) | 5,5 % |
| Dihydroxyacétone | 7 % |
| Parfum | 0,2 % |
| Eau | 5 % |

Le fluide obtenu a les mêmes caractéristiques que celui de l'exemple 1.

### Exemple 6 : Fluide autobronzant

| | |
|---|---|
| Alcool éthylique | 28,8 % |
| Vitamine E | 0,5 % |
| Butylène glycol | 15 % |
| Dioctanoate/diisononanoate de diéthylène glycol (Dermol 489 vendu par la société Alzo) | 50 % |
| Dihydroxyacétone | 3 % |
| Parfum | 0,2 % |
| Eau | 2,5 % |

Le fluide obtenu a les mêmes caractéristiques que celui de l'exemple 1.

### Exemple 7 : Fluide autobronzant

| | |
|---|---|
| Alcool éthylique | 54,3 % |
| Butylène glycol | 15 % |
| Ether stéarylique de polyoxypropylène-15 (Arlamol E vendu par la société ICI) | 20,5 % |
| Dihydroxyacétone | 5 % |
| Parfum | 0,2 % |
| Eau | 5 % |

Le fluide obtenu a les mêmes caractéristiques que celui de l'exemple 1.

### Exemple 8 : Fluide autobronzant

| | |
|---|---|
| Alcool éthylique | 34,2 % |
| Hydroxypropylcellulose | 0,1 % |
| Vitamine E | 0,5 % |
| Butylène glycol | 10 % |
| Dipropylène glycol | 7,5 % |
| Benzoate de laureth-2 (Dermol 126 vendu par la société Alzo) | 5 % |
| Dioctanoate/diisononanoate de diéthylène glycol (Dermol 489 vendu par la société Alzo) | 12,1 % |
| Ether stéarylique de polyoxypropylène-15 (Arlamol E vendu par la société ICI) | 5 % |
| Malate d'éthyl 2-hexyle | 5 % |
| Ethoxydiglycol | 10 % |
| Dihydroxyacétone | 5 % |
| Parfum | 0,6 % |
| Eau | 5 % |

Le fluide obtenu a les mêmes caractéristiques que celui de l'exemple 1.

### Exemple 9 : Fluide dépigmentant

| | |
|---|---|
| Alcool éthylique | 33,5 % |
| Hydroxypropylcellulose | 0,7 % |
| Vitamine E | 0,5 % |
| Butylène glycol | 10 % |
| Dipropylène glycol | 7,5 % |
| Dioctanoate/diisononanoate de diéthylène glycol (Dermol 489 vendu par la société Alzo) | 10 % |
| Benzoate de laureth-2 (Dermol 126 vendu par la société Alzo) | 10 % |
| Ether stéarylique de polyoxypropylène-15 (Arlamol E vendu par la société ICI) | 7,5 % |
| Pentane diol-1,2 (Hydrolite-5 vendu par la société Dragoco) | 10 % |
| Acide ascorbique | 5 % |
| Parfum | 0,3 % |
| Eau | 5 % |

Le fluide obtenu se présente sous forme d'un liquide transparent, stable, agréable à appliquer. Son application régulière permet d'éliminer les taches de la peau.

### Exemple 10 : Fluide nettoyant

| | |
|---|---|
| Alcool éthylique | 33,8 % |
| Hydroxypropylcellulose | 0,7 % |
| Butylène glycol | 10 % |
| Dipropylène glycol | 7,5 % |
| Dioctanoate/diisononanoate de diéthylène glycol (Dermol 489 vendu par la société Alzo) | 10 % |
| Benzoate de laureth-2 (Dermol 126 vendu par la société Alzo) | 10 % |
| Ether stéarylique de polyoxypropylène-15 (Arlamol E vendu par la société ICI) | 8 % |
| Pentane diol-1,2 (Hydrolite-5 vendu par la société Dragoco) | 10 % |
| Enzyme (Subtilisine SP 544 par la société Novo Nordisk) | 0,1 % |
| Eau | 9,9 % |

On obtient un fluide transparent destiné au nettoyage de la peau du visage et du corps.

Il a été réalisé un test analytique pour déterminer la stabilité de la dihydroxyacétone après conservation à 45°C pendant un certain temps pour deux compositions selon l'invention (exemples 11 et 12) et deux compositions comparatives (contre-exemples 1 et 2), la teneur en dihydroxyacétone étant à l'origine de 5 % dans toutes les compositions.

Les compositions testées ont été les suivantes :

### Exemple 11 : fluide bronzant selon l'invention

| | |
|---|---|
| Alcool éthylique | 59,3 % |
| Vitamine E | 0,5 % |
| Butylène glycol | 15 % |
| Dioctanoate/diisononanoate de diéthylène glycol (Dermol 489 vendu par la société Alzo) | 10 % |
| Dihydroxyacétone | 5 % |
| Parfum | 0,2 % |
| Eau | 10 % |

### Exemple 12 : fluide bronzant selon l'invention

| | |
|---|---|
| Alcool éthylique | 54,3 % |
| Vitamine E | 0,5 % |
| Butylène glycol | 15 % |
| Dioctanoate/diisononanoate de diéthylène glycol (Dermol 489 vendu par la société Alzo) | 15 % |
| Ether stéarylique de polyoxypropylène-15 (Arlamol E vendu par la société ICI) | 5 % |
| Dihydroxyacétone | 5 % |
| Parfum | 0,2 % |
| Eau | 5 % |

### Contre-exemple 1 (comparatif) : gel autobronzant

| | |
|---|---|
| Copolymère méthyl vinyl éther/anhydride maléique réticulé par le 1,9-décadiène (Stabileze 06 vendu par la société ISP) | 0,5 % |
| Amino-2 méthyl-2 propanol-1 | 0,25 % |
| Alcool éthylique | 45 % |
| Butylène glycol | 15 % |
| Dioctanoate/diisononanoate de diéthylène glycol (Dermol 489 vendu par la société Alzo) | 20 % |
| Huile de ricin hydrogénée PEG-60 (Cremophor RH60 vendu par la société BASF) | 0,5 % |
| Dihydroxyacétone | 5 % |
| Parfum | 0,2 % |
| Eau | 13,55 % |

Le gel du contre-exemple 1 est translucide et peu fluide. Il diffère des compositions de l'invention par une teneur en eau plus importante.

### Contre-exemple 2 (comparatif) : microémulsion autobronzante

| | |
|---|---|
| Isohexadécane (Arlamol HD vendu par la société ICI) | 44 % |
| Diméthicone (Dow corning 200 Fluid vendu par la société Dow Corning) | 18,8 % |
| Mélange de PEG-8 OE et de laurate de glycéryle (tensioactif) | 21,6 % |
| Plurol laurique (tensioactif) | 5,4 % |
| Dihydroxyacétone | 5 % |
| Parfum | 0,2 % |
| Eau | 5 % |

On obtient une microémulsion transparente qui diffère des compositions de l'invention par le fait que les huiles utilisées ne sont pas amphiphiles et par la présence de tensioactif.

Les résultats comparatifs des exemples 11 et 12 et des contre-exemples 1 et 2 sont rassemblés dans le tableau suivant :

| Nombre de jours à 45°C | Exemple 11 (invention) | Exemple 12 (invention) | Contre-exemple 1 | Contre-exemple 2 |
|---|---|---|---|---|
| 30 | 3,9 | 4,8 | 3,7 | 1,9 |
| 50 | 4,2 | 4,7 | 1,5 | 2,0 |

La quantité de dihydroxyacétone étant à T0 de 5 %, ces résultats montrent clairement que les compositions selon l'invention maintiennent plus longtemps l'activité de la dihydroxyacétone que les produits comparatifs. Il en ressort que la stabilité de la dihydroxyacétone dans les compositions selon l'invention est nettement supérieure à celle de l'art antérieur.

## Revendications

1. Support, caractérisé en ce qu'il comprend au plus 10 % en poids d'eau, au moins une huile amphiphile, au moins un polyol ou dérivé de polyol choisi parmi les glycols en C₂ à C₄, les dérivés éther-oxydes d'un glycol en C₂ à C₄ et leurs mélanges, et au moins un solvant de l'huile et de l'eau, à fonction alcool et en ce qu'il est exempt de tensioactif.

2. Support selon la revendication 1, caractérisé en ce que le solvant est choisi parmi les alcools primaires en C₂-C₈, les glycols en C₅ à C₇ et leurs mélanges.

3. Support selon la revendication 1 ou 2, caractérisé en ce qu'elle comprend de 1 à 10 % en poids d'eau.

4. Support selon l'une quelconque des revendications précédentes, caractérisé en ce qu'il est exempt de stabilisant.

5. Support selon l'une quelconque des revendications précédentes, caractérisé en ce que l'huile amphiphile est choisie parmi les esters et les éthers comportant un atome d'oxygène ayant des affinités avec l'eau et ayant un HLB de 6 à 12.

6. Support selon l'une quelconque des revendications précédentes, caractérisé en ce que l'huile amphiphile est choisie dans le groupe comprenant le benzoate de laureth-2, le benzoate de glycereth-7, le dioctanoate/diisononanoate de diéthylène glycol, l'éther stéarylique de polyoxypropylène-15, le malate d'éthyl 2-hexyle, l'adipate d'isopropyle, le copolymère de PPG-7 et d'acide succinique, le dioctanoate de néopentylglycol, et leurs mélanges.

7. Support selon l'une quelconque des revendications précédentes, caractérisé en ce que le glycol en C₂ à C₄ est choisi parmi le propylène glycol, le butylène glycol et leurs mélanges.

8. Support selon l'une quelconque des revendications précédentes, caractérisé en ce que le dérivé éther-oxyde d'un glycol en C₂ à C₄ est choisi parmi le dipropylène glycol, l'éthoxydiglycol et leurs mélanges.

9. Support selon l'une quelconque des revendications précédentes, caractérisé en ce que l'alcool primaire est l'éthanol.

10. Support selon l'une quelconque des revendications précédentes, caractérisé en ce que le glycol en C₅ à C₇ est le pentanediol-1,2.

11. Support selon l'une quelconque des revendications précédentes, caractérisé en ce que l'huile amphiphile est présente en une quantité allant de 10 à 55 % en poids par rapport au poids total du support.

12. Support selon l'une quelconque des revendications précédentes, caractérisé en ce que le polyol est présent en une quantité allant de 10 à 20 % en poids par rapport au poids total du support.

13. Support selon l'une quelconque des revendications précédentes, caractérisé en ce que le solvant est présent en une quantité allant de 5 à 60 % en poids par rapport au poids total du support.

14. Composition contenant un actif à action topique, sensible aux facteurs extérieurs et/ou à l'eau, caractérisée en ce qu'elle contient un support selon l'une quelconque des revendications précédentes.

15. Composition selon la revendication précédente, caractérisée en ce que l'actif à action topique est choisi dans le groupe comprenant les enzymes et les actifs comportant au moins une fonction hydroxyle.

16. Composition selon la revendication précédente, caractérisée en ce que l'actif comportant au moins une fonction hydroxyle est choisi dans le groupe comprenant les vitamines estérifiables, les cétones hydroxylées et leurs mélanges.

17. Composition selon la revendication 15 ou 16, caractérisée en ce que l'actif comportant au moins une fonction hydroxyle est choisi dans le groupe comprenant le rétinol, l'acide ascorbique, la dihydroxyacétone et leurs mélanges.

18. Composition selon l'une quelconque des revendications 14 à 17, caractérisée en ce qu'elle contient un actif à action topique en une quantité allant de 0,5 à 10 % en poids par rapport au poids total de la composition.

19. Composition selon l'une quelconque des revendications précédentes, caractérisée en ce qu'elle constitue une composition cosmétique et/ou dermatologique.

20. Composition selon l'une quelconque des revendications précédentes, caractérisée en ce qu'elle comprend au moins un adjuvant lipophile ou hydrophile choisi parmi les conservateurs, les antioxydants, les parfums, les filtres, les gélifiants, les séquestrants, les huiles essentielles, les matières colorantes, les actifs.

21. Utilisation de la composition selon l'une quelconque des revendications 14 à 20 pour la dépigmentation de la peau, l'actif étant l'acide ascorbique.

22. Utilisation de la composition selon l'une quelconque des revendications 14 à 20 pour améliorer l'éclat de la peau, l'actif étant l'acide ascorbique.

23. Utilisation de la composition selon l'une quelconque des revendications 14 à 20 pour préparer une pommade ou un onguent dermatologique destiné au traitement thérapeutique de l'acné, l'actif étant l'acide ascorbique et'ou le rétinol.

24. Utilisation de la composition selon l'une quelconque des revendications 14 à 20 pour colorer la peau, l'actif étant la dihydroxyacétone.

25. Composition autobronzante à base de dihydroxyacétone, caractérisée en ce qu'elle comprend au plus 10 % en poids d'eau, au moins une huile amphiphile, au moins un polyol ou dérivé de polyol choisi parmi les glycols en C₂ à C₄, les dérivés éther-oxydes d'un glycol en C₂ à C₄ et leurs mélanges, et au moins un solvant de l'huile et de l'eau, à fonction alcool.

26. Composition selon la revendication 25, caractérisée en ce que la dihydroxyacétone est présente en une quantité allant de 1 à 8 % en poids par rapport au poids total de la composition.

## Claims

1. Base characterized in that it includes not more than 10 % by weight of water, at least one amphiphilic oil, at least one polyol or polyol derivative chosen from C₂-C₄ glycols, ether derivatives of a C₂-C₄ glycol and mixtures thereof, and at least one solvent for oil and water, containing an alcohol functional group, and in that it is free from surfactant.

2. Base according to Claim 1, characterized in that the solvent is chosen from C₂-C₈ primary alcohols, C₅-C₇ glycols and mixtures thereof.

3. Base according to Claim 1 or 2, characterized in that it includes from 1 to 10 % by weight of water.

4. Base according to any one of the preceding claims, characterized in that it is free from stabilizer.

5. Base according to any one of the preceding claims, characterized in that the amphiphilic oil is chosen from esters and ethers containing an oxygen atom which have affinities with water and which have an HLB of 6 to 12.

6. Base according to any one of the preceding claims, characterized in that the amphiphilic oil is chosen from the group including laureth-2 benzoate, glycereth-7 benzoate, diethylene glycol dioctanoate/diisononanoate, polyoxypropylene-15 stearyl ether, 2-ethylhexyl malate, isopropyl adipate, the copolymer of PPG-7 and of succinic acid, neopentyl glycol dioctanoate and mixtures thereof.

7. Base according to any one of the preceding claims, characterized in that the C₂-C₄ glycol is chosen from propylene glycol, butylene glycol and mixtures thereof.

8. Base according to any one of the preceding claims, characterized in that the ether derivative of a C₂-C₄ glycol is chosen from dipropylene glycol, ethoxydiglycol and mixtures thereof.

9. Base according to any one of the preceding claims, characterized in that the primary alcohol is ethanol.

10. Base according to any one of the preceding claims, characterized in that the C₅-C₇ glycol is 1,2-pentanediol.

11. Base according to any one of the preceding claims, characterized in that the amphiphilic oil is present in a quantity ranging from 10 to 55 % by weight relative to the total weight of the base.

12. Base according to any one of the preceding claims, characterized in that the polyol or polyol derivative is present in a quantity ranging from 10 to 20 % by weight relative to the total weight of the base.

13. Base according to any one of the preceding claims, characterized in that the solvent is present in a quantity ranging from 5 to 60 % by weight relative to the total weight of the base.

14. Composition containing an active substance with topical action, sensitive to external factors and/or to water, characterized in that it contains a base according to any one of the preceding claims.

15. Composition according to the preceding claim, characterized in that the active substance with topical action is chosen from the group including enzymes and active substances containing at least one hydroxyl functional group.

16. Composition according to the preceding claim, characterized in that the active substance containing at least one hydroxyl functional group is chosen from the group including esterifiable vitamins, hydroxylated ketones and mixtures thereof.

17. Composition according to Claim 15 or 16, characterized in that the active substance containing at least one hydroxyl functional group is chosen from the group including retinol, ascorbic acid, dihydroxyacetone and mixtures thereof.

18. Composition according to any one of Claims 14 to 17, characterized in that it contains an active substance with topical action in a quantity ranging from 0.5 to 10 % by weight relative to the total weight of the composition.

19. Composition according to any one of the preceding claims, characterized in that it constitutes a cosmetic and/or dermatological composition.

20. Composition according to any one of the preceding claims, characterized in that it includes at least one lipophilic or hydrophilic adjuvant chosen from preservatives, antioxidants, perfumes, sunscreens, gelling agents, sequestrants, essential oils, colorants and active substances.

21. Use of the composition according to any one of Claims 14 to 20 for the depigmentation of the skin, the active substance being ascorbic acid.

22. Use of the composition according to any one of Claims 14 to 20 for improving skin radiance, the active substance being ascorbic acid.

23. Use of the composition according to any one of Claims 14 to 20 for preparing a salve or a dermatological ointment intended for the therapeutic treatment of acne, the active substance being ascorbic acid and/or retinol.

24. Use of the composition according to any one of Claims 14 to 20 for colouring the skin, the active substance being dihydroxyacetone.

25. Tanning composition based on dihydroxyacetone, characterized in that it includes not more than 10 % by weight of water, at least one amphiphilic oil, at least one polyol or polyol derivative chosen from C₂-C₄ glycols, ether derivatives of a C₂-C₄ glycol and mixtures thereof, and at least one solvent for oil and water, containing an alcohol functional group.

26. Composition according to Claim 25, characterized in that the dihydroxyacetone is present in a quantity ranging from 1 to 8 % by weight relative to the total weight of the composition.

## Patentansprüche

1. Träger, dadurch gekennzeichnet, daß er höchstens 10 Gew.-% Wasser, mindestens ein amphiphiles Öl, mindestens ein Polyol oder Polyolderivat, das unter den C₂₋₄-Glykolen, den Etheroxidderivaten eines C₂₋₄-Glykols und den Gemischen dieser Verbindungen ausgewählt ist, sowie mindestens ein Lösungsmittel für Öl und Wasser mit Alkoholgruppe enthält und dadurch, daß er frei von grenzflächenaktiven Stoffen ist.

2. Träger nach Anspruch 1, dadurch gekennzeichnet, daß das Lösungsmittel unter den primären C₂₋₈-Alkoholen, den C₅₋₇-Glykolen und den Gemischen dieser Verbindungen ausgewählt ist.

3. Träger nach Anspruch 1 oder 2, dadurch gekennzeichnet, daß er 1 bis 10 Gew.-% Wasser enthält.

4. Träger nach einem der vorhergehenden Ansprüche, dadurch gekennzeichnet, daß er frei von Stabilisatoren ist.

5. Träger nach einem der vorhergehenden Ansprüche, dadurch gekennzeichnet, daß das amphiphile Öl unter den Estern und Ethern ausgewählt ist, welche ein Sauuerstoffatom enthalten und welche eine Affinität gegenüber Wasser und einen HLB-Wert von 6 bis 12 aufweisen.

6. Träger nach einem der vorhergehenden Ansprüche, dadurch gekennzeichnet, daß das amphiphile Öl unter Laureth-2-benzoat, Glycereth-7-benzoat, Diethylenglykoldioctanoat/Diethylenglykoldiisononanoat, Polyoxypropylen-15-stearylether, 2-Ethylhexylmalat, Isopropyladipat, dem Copolymer von PPG-7 und Bernsteinsäure, Neopentylglykoldioctanoat und Gemischen dieser Verbindungen ausgewählt ist.

7. Träger nach einem der vorhergehenden Ansprüche, dadurch gekennzeichnet, daß das C₂₋₄-Glykol unter Propylenglykol, Butylenglykol und den Gemischen dieser Verbindungen ausgewählt ist.

8. Träger nach einem der vorhergehenden Ansprüche, dadurch gekennzeichnet, daß das Etheroxidderivat eines C₂₋₄-Glykols unter Dipropylenglykol, Ethoxydiglykol und den Gemischen dieser Verbindungen ausgewählt ist.

9. Träger nach einem der vorhergehenden Ansprüche, dadurch gekennzeichnet, daß der primäre Alkohol Ethanol ist.

10. Träger nach einem der vorhergehenden Ansprüche, dadurch gekennzeichnet, daß das C₅₋₇-Glykol 1,2-Pentandiol ist.

11. Träger nach einem der vorhergehenden Ansprüche, dadurch gekennzeichnet, daß das amphiphile Öl in einem Mengenanteil von 10 bis 55 Gew.-% vorliegt, bezogen auf das Gesamtgewicht des Trägers.

12. Träger nach einem der vorhergehenden Ansprüche, dadurch gekennzeichnet, daß das Polyol oder Polyolderivat in einem Mengenanteil von 10 bis 20 Gew.-% vorliegt, bezogen auf das Gesamtgewicht des Trägers.

13. Träger nach einem der vorhergehenden Ansprüche, dadurch gekennzeichnet, daß das Lösungsmittel in einem Mengenanteil von 5 bis 60 Gew.-% vorliegt, bezogen auf das Gesamtgewicht des Trägers.

14. Zusammensetzung, die einen Wirkstoff mit topischer Wirkung enthält, der gegenüber äußeren Faktoren und/oder Wasser empfindlich ist, dadurch gekennzeichnet, daß sie einen Träger nach einem der vorhergehenden Ansprüche enthält.

15. Zusammensetzung nach dem vorhergehenden Anspruch, dadurch gekennzeichnet, daß der Wirkstoff mit topischer Wirkung aus der Gruppe ausgewählt ist, welche die Enzyme und die Wirkstoffe mit mindestens einer Hydroxygruppe umfaßt.

16. Zusammensetzung nach dem vorhergehenden Anspruch, dadurch gekennzeichnet, daß der Wirkstoff mit mindestens einer Hydroxygruppe unter den veresterbaren Vitaminen, hydroxylierten Ketonen und Gemischen dieser Verbindungen ausgewählt ist.

17. Zusammensetzung nach Anspruch 15 oder 16, dadurch gekennzeichnet, daß der Wirkstoff mit mindestens einer Hydroxygruppe unter Retinol, Ascorbinsäure, Dihydroxyaceton und den Gemischen dieser Verbindungen ausgewählt ist.

18. Zusammensetzung nach einem der Ansprüche 14 bis 17, dadurch gekennzeichnet, daß sie einen Wirkstoff mit topischer Wirkung in einem Mengenanteil von 0,5 bis 10 Gew.-% enthält, bezogen auf das Gesamtgewicht der Zusammensetzung.

19. Zusammensetzung nach einem der vorhergehenden Ansprüche, dadurch gekennzeichnet, daß sie eine kosmetische und/oder dermatologische Zusammensetzung darstellt.

20. Zusammensetzung nach einem der vorhergehenden Ansprüche, dadurch gekennzeichnet, daß sie mindestens einen lipophilen oder hydrophilen Zusatzstoff umfaßt, der unter den Konservierungsmitteln, Antioxidantien, Parfums, Filtern, Gelbildnern, Maskierungsmitteln, etherischen Ölen, Farbstoffen und Wirkstoffen ausgewählt ist.

21. Verwendung der Zusammensetzung nach einem der Ansprüche 14 bis 20 zur Depigmentierung der Haut, wobei der Wirkstoff Ascorbinsäure ist.

22. Verwendung der Zusammensetzung nach einem der Ansprüche 14 bis 20 zur Verbesserung des strahlenden Aussehens der Haut, wobei der Wirkstoff Ascorbinsäure ist.

23. Verwendung der Zusammensetzung nach einem der Ansprüche 14 bis 20 zur Herstellung einer Salbe oder einer dermatologischen Salbe, die zur therapeutischen Behandlung von Akne bestimmt ist, wobei der Wirkstoff Ascorbinsäure und/oder Retinol ist.

24. Verwendung der Zusammensetzung nach einem der Ansprüche 14 bis 20 zum Färben der Haut, wobei der Wirkstoff Dihydroxyaceton ist.

25. Selbstbräunende Zusammensetzung auf der Basis von Dihydroxyaceton, dadurch gekennzeichnet, daß sie höchstens 10 Gew.-% Wasser, mindestens ein amphiphiles Öl, mindestens ein Polyol oder Polyolderivat, das unter den C₂₋₄-Glykolen, den Etheroxidderivaten eines C₂₋₄-Glykols und den Gemischen dieser Verbindungen ausgewählt ist, sowie mindestens ein Lösungsmittel für Öl und Wasser mit Alkoholgruppe umfaßt.

26. Zusammensetzung nach Anspruch 25, dadurch gekennzeichnet, daß das Dihydroxyaceton in einem Mengenanteil von 1 bis 8 Gew.-% vorliegt, bezogen auf das Gesamtgewicht der Zusammensetzung.
